# EUROPEAN PATENT APPLICATION

(11) **EP 3 750 998 A1**
(43) Date of publication of application: **16.12.2020**
(21) Application number: 19751736.0
(22) Date of filing: 01.02.2019
(51) Int. Cl.: C12N 15/62, C12N 15/17, C12N 15/81, C12P 21/00, C12N 1/16

(54) **CODON OPTIMIZED PRECURSOR GENE AND SIGNAL PEPTIDE GENE OF HUMAN INSULIN ANALOGUE**

(30) Priority: 09.02.2018 CN 201810131794
(71) Applicant: Jiangsu Hengrui Medicine Co., Ltd., Lianyungang, Jiangsu 222047 (CN)
(72) Inventor: WANG, Feifei, Lianyungang, Jiangsu 222047 (CN); CHEN, Lei, Lianyungang, Jiangsu 222047 (CN); WANG, Hongwei, Lianyungang, Jiangsu 222047 (CN)
(74) Representative: Sonzogni, Laura Gabriella
(86) International application number: PCT/CN2019/074384
(87) International publication number: WO 2019/154311

(57) **Abstract**

Provided is a nucleic acid molecule of a codon optimized precursor gene and signal peptide gene of a human insulin analogue. The nucleic acid molecule comprises a nucleic acid molecule encoding the precursor of the fusion insulin analogue and a nucleic acid molecule encoding the yeast secreting signal peptide α-factor. The nucleic acid molecule improves the expression of the precursor of the insulin analogue in Pichia Pastoris, and reduces the production cost of the human insulin analogue.

Sequence listing

<110>  JIANGSU HENGRUI MEDICINE CO.,LTD

<120>  CODON OPTIMIZED PRECURSOR GENE AND SIGNAL PEPTIDE GENE OF HUMAN
INSULIN ANALOGUE

<130>  719003CPCT

<150>  CN201810131794.3
<151>  2018-02-09

<160>  16

<170>  SIPOSequenceListing 1.0

<210>  1
<211>  255
<212>  DNA
<213>  Artificial Sequence

<220>
<221>  GENE
<222>  (1)..(255)
<223>  nucleic acid molecule encoding signal peptide


<400>  1
atgagatttc cttctatttt cactgctgtt ttgtttgccg cttcctctgc tttggcagct        60
ccagttaata caaccactga agatgagact gctcaaatcc cagccgaagc agttattggt       120
tactccgact tggaaggaga ttttgacgtc gctgttttac cattctctaa ttccactaat       180
aacggtctgt tgtttattaa tactaccatt gcttctatcg ccgctaagga ggaaggtgtg       240
tccctcgaga aaaga                                                        255

<210>  2
<211>  85
<212>  PRT
<213>  Artificial Sequence

<220>
<221>  PEPTIDE
<222>  (1)..(85)
<223>  signal peptide


<400>  2
Met Arg Phe Pro Ser Ile Phe Thr Ala Val Leu Phe Ala Ala Ser Ser
1               5                   10                  15
Ala Leu Ala Ala Pro Val Asn Thr Thr Thr Glu Asp Glu Thr Ala Gln
            20                  25                  30
Ile Pro Ala Glu Ala Val Ile Gly Tyr Ser Asp Leu Glu Gly Asp Phe
        35                  40                  45
Asp Val Ala Val Leu Pro Phe Ser Asn Ser Thr Asn Asn Gly Leu Leu
    50                  55                  60
Phe Ile Asn Thr Thr Ile Ala Ser Ile Ala Ala Lys Glu Glu Gly Val
65                  70                  75                  80
Ser Leu Glu Lys Arg
                85

<210>  3
<211>  162
<212>  DNA
<213>  Homo sapiens

<220>
<221>  GENE
<222>  (1)..(162)
<223>  nucleic acid molecule encoding human insulin analogue precursor


<400>  3
ttcgtcaacc agcacttgtg tggttcccat ttggttgagg ctctgtactt ggtctgtgga      60
gaaagaggtt tcttttacac cccaaaggct gctaagggta tcgttgagca atgttgcacc     120
tctatttgtt ccctgtatca gttggaaaac tactgcaact aa                        162

<210>  4
<211>  53
<212>  PRT
<213>  Homo sapiens

<220>
<221>  PEPTIDE
<222>  (1)..(53)
<223>  human insulin analogue precursor


<400>  4
Phe Val Asn Gln His Leu Cys Gly Ser His Leu Val Glu Ala Leu Tyr
1               5                   10                  15
Leu Val Cys Gly Glu Arg Gly Phe Phe Tyr Thr Pro Lys Ala Ala Lys
            20                  25                  30
Gly Ile Val Glu Gln Cys Cys Thr Ser Ile Cys Ser Leu Tyr Gln Leu
        35                  40                  45
Glu Asn Tyr Cys Asn
    50

<210>  5
<211>  18
<212>  DNA
<213>  Artificial Sequence

<220>
<221>  GENE
<222>  (1)..(18)
<223>  nucleic acid molecule encoding spacer peptide


<400>  5
gaagaaggtg aaccaaag                                                    18

<210>  6
<211>  180
<212>  DNA
<213>  Homo sapiens

<400>  6
gaagaaggtg aaccaaagtt cgtcaaccag cacttgtgtg gttcccattt ggttgaggct      60
ctgtacttgg tctgtggaga aagaggtttc ttttacaccc caaaggctgc taagggtatc     120
gttgagcaat gttgcacctc tatttgttcc ctgtatcagt tggaaaacta ctgcaactaa     180

<210>  7
<211>  59
<212>  PRT
<213>  Homo sapiens

<400>  7

Glu Glu Gly Glu Pro Lys Phe Val Asn Gln His Leu Cys Gly Ser His
1               5                   10                  15
Leu Val Glu Ala Leu Tyr Leu Val Cys Gly Glu Arg Gly Phe Phe Tyr
            20                  25                  30
Thr Pro Lys Ala Ala Lys Gly Ile Val Glu Gln Cys Cys Thr Ser Ile
        35                  40                  45
Cys Ser Leu Tyr Gln Leu Glu Asn Tyr Cys Asn
    50                  55

<210>  8
<211>  435
<212>  DNA
<213>  Homo sapiens

<400>  8
atgagatttc cttctatttt cactgctgtt ttgtttgccg cttcctctgc tttggcagct      60
ccagttaata caaccactga agatgagact gctcaaatcc cagccgaagc agttattggt     120
tactccgact tggaaggaga ttttgacgtc gctgttttac cattctctaa ttccactaat     180
aacggtctgt tgtttattaa tactaccatt gcttctatcg ccgctaagga ggaaggtgtg     240
tccctcgaga aaagagaaga aggtgaacca aagttcgtca accagcactt gtgtggttcc     300
catttggttg aggctctgta cttggtctgt ggagaaagag gtttctttta caccccaaag     360
gctgctaagg gtatcgttga gcaatgttgc acctctattt gttccctgta tcagttggaa     420
aactactgca actaa                                                      435

<210>  9
<211>  144
<212>  PRT
<213>  Homo sapiens

<400>  9
Met Arg Phe Pro Ser Ile Phe Thr Ala Val Leu Phe Ala Ala Ser Ser
1               5                   10                  15
Ala Leu Ala Ala Pro Val Asn Thr Thr Thr Glu Asp Glu Thr Ala Gln
            20                  25                  30
Ile Pro Ala Glu Ala Val Ile Gly Tyr Ser Asp Leu Glu Gly Asp Phe
        35                  40                  45
Asp Val Ala Val Leu Pro Phe Ser Asn Ser Thr Asn Asn Gly Leu Leu
    50                  55                  60
Phe Ile Asn Thr Thr Ile Ala Ser Ile Ala Ala Lys Glu Glu Gly Val
65                  70                  75                  80
Ser Leu Glu Lys Arg Glu Glu Gly Glu Pro Lys Phe Val Asn Gln His
                85                  90                  95
Leu Cys Gly Ser His Leu Val Glu Ala Leu Tyr Leu Val Cys Gly Glu
            100                 105                 110
Arg Gly Phe Phe Tyr Thr Pro Lys Ala Ala Lys Gly Ile Val Glu Gln
        115                 120                 125
Cys Cys Thr Ser Ile Cys Ser Leu Tyr Gln Leu Glu Asn Tyr Cys Asn
    130                 135                 140

<210>  10
<211>  180
<212>  DNA
<213>  Artificial Sequence

<220>
<221>  misc_feature
<222>  (1)..(18)
<223>  nucleic acid molecule encoding spacer peptide


<400>  10
gaagaaggtg aaccaaagtt cgttaaccaa cacttgtgcg gttcccactt ggttgaagct      60
ttgtacttgg tttgcggtga aagaggtttc ttctacactc ctaaggctgc taagggtatt     120

gtcgaacaat gctgtacctc catctgctcc ttgtaccaat tggaaaacta ctgcaactaa 180

<210> 11
<211> 180
<212> DNA
<213> Artificial Sequence

<220>
<221> misc_feature
<222> (1)..(18)
<223> nucleic acid encoding spacer peptide


<400> 11
gaagaaggtg aaccaaagtt tgttaaccaa catttgtgtg gttctcattt ggttgaagct 60
ttgtacttgg tttgtggtga aagaggtttc ttctacactc caaaggctgc taagggtatt 120
gttgaacaat gttgtacttc tatttgttct ttgtaccaat tggaaaacta ctgtaactaa 180

<210> 12
<211> 255
<212> DNA
<213> Artificial Sequence

<220>
<221> GENE
<222> (1)..(255)
<223> nucleic acid molecule encoding a-factor


<400> 12
atgagatttc cttcaatttt tactgcagtt ttattcgcag catcctccgc attagctgct 60
ccagtcaaca ctacaacaga agatgaaacg gcacaaattc cggctgaagc tgtcatcggt 120
tactcagatt tagaagggga tttcgatgtt gctgttttgc cattttccaa cagcacaaat 180
aacgggttat tgtttataaa tactactatt gccagcattg ctgctaaaga agaaggggta 240
tctctcgaga aaaga 255

<210> 13
<211> 435
<212> DNA
<213> Artificial Sequence

<220>
<221> GENE
<222> (1)..(435)
<223> nucleic acid encoding a-factor + nucleic acid encoding insulin
analogue precursor


<400> 13
atgagatttc cttcaatttt tactgcagtt ttattcgcag catcctccgc attagctgct 60
ccagtcaaca ctacaacaga agatgaaacg gcacaaattc cggctgaagc tgtcatcggt 120
tactcagatt tagaagggga tttcgatgtt gctgttttgc cattttccaa cagcacaaat 180
aacgggttat tgtttataaa tactactatt gccagcattg ctgctaaaga agaaggggta 240
tctctcgaga aaagagaaga aggtgaacca aagttcgtca accagcactt gtgtggttcc 300
catttggttg aggctctgta cttggtctgt ggagaaagag gtttctttta caccccaaag 360
gctgctaagg gtatcgttga gcaatgttgc acctctattt gttccctgta tcagttggaa 420
aactactgca actaa 435

<210> 14
<211> 435
<212> DNA
<213> Artificial Sequence

<220>
<221>  GENE
<222>  (1)..(435)
<223>  nucleic acid encoding a-factor + nucleic acid encoding insulin
analogue precursor

<400>  14
atgagatttc cttcaatttt tactgcagtt ttattcgcag catcctccgc attagctgct      60
ccagtcaaca ctacaacaga agatgaaacg gcacaaattc cggctgaagc tgtcatcggt     120
tactcagatt tagaagggga tttcgatgtt gctgttttgc cattttccaa cagcacaaat     180
aacgggttat tgtttataaa tactactatt gccagcattg ctgctaaaga agaaggggta     240
tctctcgaga aaagagaaga aggtgaacca aagttcgtta accaacactt gtgcggttcc     300
cacttggttg aagctttgta cttggtttgc ggtgaaagag gtttcttcta cactcctaag     360
gctgctaagg gtattgtcga acaatgctgt acctccatct gctccttgta ccaattggaa     420
aactactgca actaa                                                      435

<210>  15
<211>  435
<212>  DNA
<213>  Artificial Sequence

<220>
<221>  GENE
<222>  (1)..(435)
<223>  nucleic acid encoding a-factor + nucleic acid encoding insulin
analogue precursor

<400>  15
atgagatttc cttctatttt cactgctgtt ttgtttgccg cttcctctgc tttggcagct      60
ccagttaata caaccactga agatgagact gctcaaatcc cagccgaagc agttattggt     120
tactccgact tggaaggaga ttttgacgtc gctgttttac cattctctaa ttccactaat     180
aacggtctgt tgtttattaa tactaccatt gcttctatcg ccgctaagga ggaaggtgtg     240
tccctcgaga aaagagaaga aggtgaacca aagttcgtta accaacactt gtgcggttcc     300
cacttggttg aagctttgta cttggtttgc ggtgaaagag gtttcttcta cactcctaag     360
gctgctaagg gtattgtcga acaatgctgt acctccatct gctccttgta ccaattggaa     420
aactactgca actaa                                                      435

<210>  16
<211>  6
<212>  PRT
<213>  Artificial Sequence

<220>
<221>  PEPTIDE
<222>  (1)..(6)
<223>  spacer peptide

<400>  16
Glu Glu Gly Glu Pro Lys
1               5

## Description

### FIELD OF THE INVENTION

The present invention relates to a codon-optimized human insulin analogue precursor gene and a codon-optimized α-factor signal peptide gene, and provides a method for expressing the human insulin analogue precursor gene.

### BACKGROUND OF THE INVENTION

Human insulin is a polypeptide consisting of 51 amino acids and comprises two chains, Chain A and Chain B, respectively. The main efficiency of insulin is to regulate glucose metabolism. Insulin intervention is the most direct and effective method as an alternative or supplementary treatment for diabetes. Insulin also has effect on promoting synthesis of fat, inhibiting decomposition of fat, and reducing the production of ketone body, thereby it is also used to correct various symptoms of insulin-related ketosis and acidosis.

Insulin was previously extracted from the pancreas of pigs, bovine and other animals, but these products are structurally different from human insulin, so they have immunogenicity. Since genetically recombinant technology for production of human insulin was developed by Eli Lilly & Co. USA and Novo Nordisk Denmark successively, in the early and mid-1980s, genetically expressing human insulin and its analogues became the main means in the industry. However, it is extremely inconvenient for patients who need to be injected frequently with human insulin due to the short-term effect of insulin. Therefore, efforts have been made to obtain insulin analogues and derivatives with long-term effect in human. Among them, modification of human insulin or analogue thereof with acylated group is an effective method for increasing the half-life thereof. A human insulin analogue was disclosed in WO2018024186, in which position B29 was substituted with a long chain fatty acid and the amino acid at position B30 was deleted, and the structure and biological activity of the human insulin analogue were disclosed. An insulin analogue having a 14-acyl side chain linked to position B29 and an amino acid deletion at position B30, and a preparation thereof were disclosed in WO9507931. A human insulin analogue in which B29 position was substituted with a glutamic acid and a long chain fatty acid, and the amino acid at position B30 was deleted, was disclosed in WO2005012347. At present, the most commonly used expression systems for expressing human insulin and analogue thereof are *Escherichia coli, Saccharomyces Cerevisiae* and *Pichia Pastoris,* among which, human insulin and analogue thereof is expressed in the form of inclusion body in *E. coli,* and cleavage and renaturation of inclusion body are necessary, which makes the process cumbersome and low-yield; *S*. *cerevisiae* and *P. pastoris* have the advantages due to the ease of operation, ease of cultivation, modification of foreign protein, and the capability of secretion expression, and so forth. However, the secretion efficiency for *Saccharomyces Cerevisiae* is low and the strain for expression is not stable. By contrast, *Pichia Pastoris* is an expression system more widely used in industrial production of recombinant proteins. Industrially, the fermentation yield during the production process is a key factor in controlling the production cost. Due to the large demand in commercial insulin, in Novo Nordisk, a major producer, the scale of the tank reaches dozens of tons for production in yeast expression system, which involves high requirements for both plant and equipment and finally results in high cost. Thus, it is of great significance to increase the fermentation yield of human insulin and analogue thereof in industrial production.

Genetic codon is a triplet code consisting of three adjacent bases on the messenger ribonucleic acid (mRNA). There are 64 types of genetic codons. However, frequency of codon usage differs for different organisms, even for different protein-coding genes of the same organism, i.e., there is codon preference. Codons of foreign genes mainly affect gene expression at the translational level. There are many literatures showing that codon optimization has significant effect on increasing the expression of foreign proteins in *Pichia Pastoris.* Exogenous genes are expressed in *Pichia Pastoris* in the form of intracellular expression and secretory expression. For the latter, signal peptides are required to direct the secretion of products expressed by exogenous genes. Currently, the most commonly used signal peptide is derived from α-factor signal peptide of *Saccharomyces Cerevisiae,* and the nucleotide sequence thereof is also derived from *Saccharomyces Cerevisiae.* α-factor signal peptide nucleotide sequence optimized for *Pichia Pastoris* has not been reported so far. There are many related literatures on codon optimization of insulin precursors, for example, an optimized human insulin precursor gene sequence and its expression in *Pichia Pastoris* by Gurramkonda et al. (Gurramkonda et al. Application of simple fed-batch technique to high-level secretory production of insulin precursor using Pichia pastoris with subsequent purification and conversion to human insulin. Microbial Cell Factories, 2010, 9:31), and patent publication WO1998028429 discloses a gene sequence expressing a human insulin analogue precursor, and the insulin precursor amino acid sequence encoded by the gene is EEGEPK-B(1-29)-AAK-A(1-21), wherein EEGEPK is an N-terminal extension of the insulin precursor, referred to as spacer peptide or leader peptide, which is capable of protecting the N-terminus of the insulin precursor from the hydrolysis via yeast protease, and is capable of improving the expression efficiency of the insulin precursor; B (1-29) is human insulin Chain B with deletion of B30 threonine; A (1-21) is the amino acid sequence of human insulin Chain A; AAK is a linker peptide linking Chain B to Chain A, also referred to as C peptide.

In order to further increase the yield of human insulin and its analogue precursors, the inventors optimized the insulin analogue precursor gene and the α-factor signal peptide gene for secretory expression in *Pichia Pastoris* according to the codon preference in *Pichia Pastoris.* Our results show that the yield of the human insulin analogue precursor was increased by almost two fold by the codon-optimized gene expression according to the present invention, when compared with the human insulin analogue precursor gene known in the prior art (as a control). The cost of industrial production of human insulin and its analogues will be greatly reduced in the late stage.

### SUMMARY OF THE INVENTION

In some embodiments of the invention, provides a nucleic acid molecule comprising the following structure:

5'- (PS)ₐ - (SP)_{b} - (LS)_{c} - GE - (P'S)_{d} - 3',

wherein PS is a nucleic acid molecule encoding a processing site, a is 0 or 1;
SP is a nucleic acid molecule encoding signal peptide, b is 0 or 1;
LS is a nucleic acid molecule encoding spacer peptide, c is 0 or 1;
GE is a nucleic acid molecule encoding polypeptide of interest; and
P'S is a nucleic acid molecule encoding processing site, and d is 0 or 1.

In some embodiments, provides a nucleic acid molecule comprising the following structure:

5'- (PS)ₐ - (SP)_{b} - (LS)_{c} - GE - (P'S)_{d}- 3',

wherein PS is a nucleic acid molecule encoding processing site, a is 0 or 1;
SP is a nucleic acid molecule encoding signal peptide, b is 1;
LS is a nucleic acid molecule encoding spacer peptide, c is 1;
GE is a nucleic acid molecule encoding polypeptide of interest; and
P'S is a nucleic acid molecule encoding processing site, and d is 0 or 1.

In some embodiments, the nucleic acid molecule encoding signal peptide comprises the sequence shown as SEQ ID NO:1.

In some embodiments, the polypeptide of interest is a human insulin analogue precursor polypeptide; the nucleic acid molecule encoding the human insulin analogue precursor polypeptide comprises the sequence shown as SEQ ID NO:3.

In some embodiments, the nucleic acid sequence of the nucleic acid molecule encoding signal peptide (SP) is shown as SEQ ID NO: 1, and the amino acid sequence thereof is shown as SEQ ID NO: 2:

ATGAGATTTCCTTCTATTTTCACTGCTGTTTTGTTTGCCGCTTCCTCTGCTTT
GGCAGCTCCAGTTAATACAACCACTGAAGATGAGACTGCTCAAATCCCAGC
CGAAGCAGTTATTGGTTACTCCGACTTGGAAGGAGATTTTGACGTCGCTGT
TTTACCATTCTCTAATTCCACTAATAACGGTCTGTTGTTTATTAATACTACCAT
TGCTTCTATCGCCGCTAAGGAGGAAGGTGTGTCCCTCGAGAAAAGA
SEQ ID NO:1;

MRFPSIFTAVLFAASSALAAPVNTTTEDETAQIPAEAVIGYSDLEGDFDVAVLPF
SNSTNNGLLFINTTIASIAAKEEGVSLEKR
SEQ ID NO:2;

In some embodiments, the nucleic acid molecule encoding polypeptide of interest (GE) may be a nucleic acid molecule encoding human insulin analogue precursor, wherein the human insulin analogue precursor may be human insulin with a deletion of threonine at position B30. The nucleic acid molecule sequence of the human insulin analogue precursor is shown as SEQ ID NO:3, and the amino acid sequence thereof is shown as SEQ ID NO: 4: 
TTCGTCAACCAGCACTTGTGTGGTTCCCATTTGGTTGAGGCTCTGTACTTGG
TCTGTGGAGAAAGAGGTTTCTTTTACACCCCAAAGGCTGCTAAGGGTATCG
TTGAGCAATGTTGCACCTCTATTTGTTCCCTGTATCAGTTGGAAAACTACTG
CAACTAA
SEQ ID NO: 3;
 FVNQHLCGSHLVEALYLVCGERGFFYTPKAAKGIVEQCCTSICSLYQLENYCN
SEQ ID NO:4.

In some embodiments, positions 88-96 of the nucleic acid molecule encoding human insulin analogue precursor (i.e., GCTGCTAAG) presents a nucleic acid molecule encoding linker peptide (also referred to as C-peptide), which may be substituted with the following sequence, including but not limited to: GCCGCTAAG, GCTGCCAAG, GCTGCTAAA, GCCGCCAAG.

In some embodiments, the sequence of the nucleic acid molecule (LS) encoding spacer peptide is shown as SEQ ID NO: 5.
gaagaaggtgaaccaaag
SEQ ID NO:5.

In some embodiments, the PS and/or P'S are nucleic acid molecules encoding restriction site.

Preferably, PS is a nucleic acid molecule encoding EcoR I restriction site, and/or P'S is a nucleic acid molecule encoding Not I restriction site.

In some embodiments, provides a nucleic acid molecule capable of expressing human insulin analogue precursor, wherein the nucleic acid molecule comprises a nucleic acid molecule encoding spacer peptide and a nucleic acid molecule encoding human insulin analogue precursor, and is capable of expressing human insulin analogue precursor, after recombination with a vector comprising a signal peptide.

In some embodiments, the amino acid sequence of the human insulin analogue precursor encoded by the human insulin analogue precursor nucleic acid molecule is as follows:

EEGEPK-B(1-29)-AAK-A(1-21)

wherein "EEGEPK (SEQ ID NO: 16)" may be a N-terminal extension of the insulin precursor, referred to as spacer peptide or leader peptide; "B(1-29)" may be a human insulin Chain B with a deletion of threonine on position B30. "A(1-21)" may be a human insulin Chain A amino acid sequence, and "AAK" is a linker peptide linking Chain B to Chain A, also referred to as C peptide.

In some embodiments, the sequence of the human insulin analogue precursor nucleic acid molecule may be shown as SEQ ID NO: 6, and the amino acid sequence thereof is shown as SEQ ID NO:7:

GAAGAAGGTGAACCAAAGTTCGTCAACCAGCACTTGTGTGGTTCCCATTTG
GTTGAGGCTCTGTACTTGGTCTGTGGAGAAAGAGGTTTCTTTTACACCCCA
AAGGCTGCTAAGGGTATCGTTGAGCAATGTTGCACCTCTATTTGTTCCCTGT
ATCAGTTGGAAAACTACTGCAACTAA
SEQ ID NO:6;

EEGEPKFVNQHLCGSHLVEALYLVCGERGFFYTPKAAKGIVEQCCTSICSLYQL
ENYCN
SEQ ID NO:7.

In some embodiments, another nucleic acid molecule capable of expressing human insulin analogue precursor is provided. The nucleic acid molecule sequence comprises a signal peptide sequence, a spacer peptide sequence, and a sequence encoding human insulin analogue precursor, and the nucleic acid molecule is capable of expressing human insulin analogue precursor after recombination with a vector comprising no signal peptide.

In some embodiments, the nucleic acid sequence of the nucleic acid molecule expressing human insulin analogue precursor is shown as SEQ ID NO: 8, and the encoded amino acid sequence is shown as SEQ ID NO:9:

ATGAGATTTCCTTCTATTTTCACTGCTGTTTTGTTTGCCGCTTCCTCTGCTTT
GGCAGCTCCAGTTAATACAACCACTGAAGATGAGACTGCTCAAATCCCAGC
CGAAGCAGTTATTGGTTACTCCGACTTGGAAGGAGATTTTGACGTCGCTGT
TTTACCATTCTCTAATTCCACTAATAACGGTCTGTTGTTTATTAATACTACCAT
TGCTTCTATCGCCGCTAAGGAGGAAGGTGTGTCCCTCGAGAAAAGAGAAG
AAGGTGAACCAAAGTTCGTCAACCAGCACTTGTGTGGTTCCCATTTGGTTG
AGGCTCTGTACTTGGTCTGTGGAGAAAGAGGTTTCTTTTACACCCCAAAGG
CTGCTAAGGGTATCGTTGAGCAATGTTGCACCTCTATTTGTTCCCTGTATCA
GTTGGAAAACTACTGCAACTAA
SEQ ID NO:8;

MRFPSIFTAVLFAASSALAAPVNTTTEDETAQIPAEAVIGYSDLEGDFDVAVLPF
SNSTNNGLLFINTTIASIAAKEEGVSLEKREEGEPKFVNQHLCGSHLVEALYLV
CGERGFFYTPKAAKGIVEQCCTSICSLYQLENYCN
SEQ ID NO:9.

In some embodiments, the nucleic acid molecule expressing human insulin analogue precursor may also comprise restriction site(s), preferably the restriction site(s) is (are) EcoR I restriction site and/or Not I restriction site.

In some embodiments, a vector capable of being expressed in a eukaryotic or prokaryotic cell is provided, which is capable of expressing human insulin analogue precursor in a prokaryotic or eukaryotic cell via secretory expression.

In some embodiments, a host cell is also provided. Preferably, the host cell is yeast, more preferably *Pichia,* which is capable of expressing human insulin analogue precursor via secretory expression.

In some embodiments, a method for preparing a human insulin analogue is also provided, comprising utilizing the nucleic acid molecule, vector, and/or host cell as described above.

The method may further comprise the following steps:
1) expressing a human insulin analogue precursor in a eukaryotic cell by a nucleic acid molecule encoding the human insulin analogue precursor;
2) obtaining a human insulin analogue by enzymatically digesting the human insulin analogue precursor, the nucleic acid molecule encoding the human insulin analogue precursor may be shown as SEQ ID NO: 6, and the insulin analogue precursor may be enzymatically digested by using the methods well known to those skilled in the art.

In some embodiments, step 1) comprises expressing the human insulin analogue precursor by an expression vector comprising a signal peptide sequence, and said signal peptide sequence is shown as SEQ ID NO: 1.

In some embodiments, the human insulin analogue is a human insulin with deletion of B30, and the human insulin analogue is further substituted with an acylated group.

In some embodiments, within said human insulin with deletion of B30, the lysine at position B29 is substituted by the acylated group.

Preferably, the product obtained after the above substitution is lysine B29 (N^{ε}-(N^{α}-hexadecane fatty diacid-L-lysine-N^{ε}-oxobutylyl)) des(B30) human insulin.

### Abbreviations and terms

"Codon optimization" refers to the synthesis of genes with preferred codons instead of codons with low frequency or rare codons according to the rule of codon preference for host cells.

"Control 1" is shown as SEQ ID NO: 10 below, wherein a nucleic acid molecule encoding "EEGEPK" (GAAGAAGGTGAACCAAAG, double underlined) is linked to a nucleic acid molecule encoding the insulin precursor gene taught by WO1998028429:

GAAGAAGGTGAACCAAAGTTCGTTAACCAACACTTGTGCGGTTCCCACTT
GGTTGAAGCTTTGTACTTGGTTTGCGGTGAAAGAGGTTTCTTCTACACTCCT
AAGGCTGCTAAGGGTATTGTCGAACAATGCTGTACCTCCATCTGCTCCTTGT
ACCAATTGGAAAACTACTGCAACTAA
SEQ ID NO:10.

"Control 2" is shown as SEQ ID NO: 11 below, wherein a nucleic acid molecule encoding "EEGEPK" (double underlined) is linked to a nucleic acid molecule encoding the optimized insulin precursor gene taught by Gurramkonda et al. (Microbial Cell Factories, 2010, 9:31):

GAAGAAGGTGAACCAAAGTTTGTTAACCAACATTTGTGTGGTTCTCATTTG
GTTGAAGCTTTGTACTTGGTTTGTGGTGAAAGAGGTTTCTTCTACACTCCA
AAGGCTGCTAAGGGTATTGTTGAACAATGTTGTACTTCTATTTGTTCTTTGTA
CCAATTGGAAAACTACTGTAACTAA
SEQ ID NO: 11.

"IP-S" is a nucleic acid molecule corresponding to an insulin precursor gene after codon optimization.

"a-factor" is a nucleic acid molecule corresponding to the α-factorsignal peptide gene comprised in pPIC9K Expression Vector provided by Invitrogen, which is derived from *Saccharomyces Cerevisiae.*

"a-factor-S" is a nucleic acid molecule corresponding to a codon-optimized α-factorsignal peptide gene.

"Vector" includes nucleic acid molecule, which is capable of transporting another nucleic acid to which it is linked, including but not limited to, plasmid and viral vector. Some vectors are capable of autonomously replicating in the host cell into which they are introduced, while others can be integrated into the genome of the host cell upon introduction into the host cell and thereby being replicated along with the host genome. In addition, some vectors are capable of directing the expression of genes operably linked thereto, and such vectors are referred to herein as "recombinant expression vectors" (or simply as "expression vectors"). Traditional vectors are well known in the art.

"Polypeptide of interest" is a polypeptide that can be expressed in yeast, including but not limited to enzyme, antibody, interferon, insulin, interleukin, and the like, and variant, precursor, intermediate thereof. For example, polypeptide of interest may be insulin precursor.

"Cell" and "host cell" may be interchangeably used.

"Polynucleotide molecule", "nucleic acid molecule" may be interchangeably used and the sequence thereof may be DNA sequence.

### DETAILED DESCRIPTION OF THE DISCLOSURE

The following examples are provided to further illustrate the invention, but are not intended to limit the scope of the invention.

The vectors, host bacteria and culture media used in the examples of the present invention were purchased from Invitrogen. pPIC9K, a Pichia Pastoris expression vector, contains an alcohol oxidase AOX1 promoter, which can be induced by methanol, and the vector also contains α-factorsignal peptide sequence and is capable of expressing foreign proteins via secretory expression. pPIC3.5K, another *Pichia Pastoris* expression vector, contains alcohol oxidase AOX1 promoter, which can be induced by methanol, and the vector does not contain α-factorsignal peptide sequence. *Pichia Pastoris* GS115 strain was used as host bacteria. The formulation of the culture medium was provided by the *Pichia Pastoris* manual.

### Example 1 Construction of Recombinant Expression Vector for Insulin Precursor

EcoR I and Not I restriction sites were added into the 5'- and 3'-ends of Control 1 (SEQ ID NO: 10), Control 2 (SEQ ID NO: 11) and IP-S (SEQ ID NO: 6), respectively and synthesized by Nanjing Kingsray Biotech Co., Ltd.. The synthesized nucleic acid molecule sequence was ligated into T vector.

GAAGAAGGTGAACCAAAGTTCGTCAACCAGCACTTGTGTGGTTCCCATTTG
GTTGAGGCTCTGTACTTGGTCTGTGGAGAAAGAGGTTTCTTTTACACCCCA
AAGGCTGCTAAGGGTATCGTTGAGCAATGTTGCACCTCTATTTGTTCCCTGT
ATCAGTTGGAAAACTACTGCAACTAA
SEQ ID NO:6;

GAAGAAGGTGAACCAAAGTTCGTTAACCAACACTTGTGCGGTTCCCACTT
GGTTGAAGCTTTGTACTTGGTTTGCGGTGAAAGAGGTTTCTTCTACACTCCT
AAGGCTGCTAAGGGTATTGTCGAACAATGCTGTACCTCCATCTGCTCCTTGT
ACCAATTGGAAAACTACTGCAACTAA
SEQ ID NO:10;

GAAGAAGGTGAACCAAAGTTTGTTAACCAACATTTGTGTGGTTCTCATTTG
GTTGAAGCTTTGTACTTGGTTTGTGGTGAAAGAGGTTTCTTCTACACTCCA
AAGGCTGCTAAGGGTATTGTTGAACAATGTTGTACTTCTATTTGTTCTTTGTA
CCAATTGGAAAACTACTGTAACTAA
SEQ ID NO:11.

The T vector carrying the insulin precursor nucleic acid molecule and the expression vector pPIC9K were digested with both EcoR I and Not I, and then the target fragment and the vector fragment were separately recovered by a Gel Recovery Kit. After purification of the digested fragments, and the target fragment was ligated to the vector pPIC9K with T4 ligase.

The above-mentioned ligation solution was transformed into E. coli TOP10 competent cells, and plated onto a plate with ampicillin resistance. After cultivation, the bacterial colony was picked, and the plasmid was extracted and verified by digestion with both restriction enzymes. Three recombinant expression vectors comprising Control 1, Control 2 and IP-S sequence respectively, were finally obtained.

### Example 2 Expression of insulin precursor by Pichia Pastoris recombinant strain

The three recombinant expression vectors constructed in Example 1 were transformed into Pichia Pastoris GS115 respectively, the recombinant strains expressing Control 1 and Control 2 were used as control strains, and the recombinant strain expressing IP-S was used as test strain.

The colonies from the three recombinant bacteria were inoculated into 5 mL YPD medium, and cultivated at a constant temperature of 30°C while shaking at 250 rpm, until the value of OD₆₀₀ reached about 10 (16-18 hours). The cells were collected and resuspended in 50 mL BMGY medium, and cultivated overnight at a constant temperature of 30°C while shaking at 250 rpm, until the value of OD₆₀₀ reached about 30. The cells were collected by centrifuging at 1500 rpm for 5 minutes and resuspended in 25 mL of BMMY medium. 1/200 volume of methanol (final concentration of 0.5%) was added into the medium, and cultivated at a constant temperature of 30°C while shaking at 250 rpm for 96 hours, while 1/200 volume of methanol was supplemented every 24 hours. Upon the expression was finished, the supernatant was obtained after centrifugation at 10,000 rpm. The yield of the insulin precursor comprised in the supernatant was measured by HPLC, and converted into a percentage relative to the expression amount of the insulin precursor expressed by the control strain. The percentage of expression level of the insulin precursor is shown as Table 1.

**Table 1**

| Bacterial strain | vector | Signal peptide | Expression Gene | Percentage of yield (%) |
|---|---|---|---|---|
| Control bacterium | pPIC9K | α-factor | Control 1 | 100 |
| Control b acterium | pPIC9K | α-factor | Control 2 | 125 |
| Test bacterium | pPIC9K | a-factor | IP-S | 225 |

The data in Table 1 shows that the amount of insulin precursor expressed by the optimized insulin precursor gene was increased by 1.8 to 2.25 times compared to those in the two control groups. It can be seen that the optimized insulin precursor gene has superior expression efficiency and can significantly improve the yield of the expressed insulin precursor.

### Example 3 Construction of recombinant expression vectors comprising insulin precursor gene fused to different α-factors

Nucleic acid molecules shown as SEQ ID NO: 13 and SEQ ID NO: 8, in which α-factor(SEQ ID NO: 12) and α-factor-S (SEQ ID NO: 1) were separately fused to the site in the upstream of IP-S, were synthesized. EcoR I and Not I restriction sites were also incorporated at both 5' and 3' ends of the synthesized nucleic acid molecules. The synthesized nucleic acid molecules were ligated to the T vector.

ATGAGATTTCCTTCAATTTTTACTGCAGTTTTATTCGCAGCATCCTCCGCATT
AGCTGCTCCAGTCAACACTACAACAGAAGATGAAACGGCACAAATTCCGG
CTGAAGCTGTCATCGGTTACTCAGATTTAGAAGGGGATTTCGATGTTGCTGT
TTTGCCATTTTCCAACAGCACAAATAACGGGTTATTGTTTATAAATACTACTA
TTGCCAGCATTGCTGCTAAAGAAGAAGGGGTATCTCTCGAGAAAAGA
SEQ ID NO:12

ATGAGATTTCCTTCTATTTTCACTGCTGTTTTGTTTGCCGCTTCCTCTGCTTT
GGCAGCTCCAGTTAATACAACCACTGAAGATGAGACTGCTCAAATCCCAGC
CGAAGCAGTTATTGGTTACTCCGACTTGGAAGGAGATTTTGACGTCGCTGT
TTTACCATTCTCTAATTCCACTAATAACGGTCTGTTGTTTATTAATACTACCAT
TGCTTCTATCGCCGCTAAGGAGGAAGGTGTGTCCCTCGAGAAAAGA
SEQ ID NO:1

ATGAGATTTCCTTCAATTTTTACTGCAGTTTTATTCGCAGCATCCTCCGCATT
AGCTGCTCCAGTCAACACTACAACAGAAGATGAAACGGCACAAATTCCGG
CTGAAGCTGTCATCGGTTACTCAGATTTAGAAGGGGATTTCGATGTTGCTGT
TTTGCCATTTTCCAACAGCACAAATAACGGGTTATTGTTTATAAATACTACTA
TTGCCAGCATTGCTGCTAAAGAAGAAGGGGTATCTCTCGAGAAAAGAGAA
GAAGGTGAACCAAAGTTCGTCAACCAGCACTTGTGTGGTTCCCATTTGGTT
GAGGCTCTGTACTTGGTCTGTGGAGAAAGAGGTTTCTTTTACACCCCAAAG
GCTGCTAAGGGTATCGTTGAGCAATGTTGCACCTCTATTTGTTCCCTGTATC
AGTTGGAAAACTACTGCAACTAA
SEQ ID NO:13

ATGAGATTTCCTTCTATTTTCACTGCTGTTTTGTTTGCCGCTTCCTCTGCTTT
GGCAGCTCCAGTTAATACAACCACTGAAGATGAGACTGCTCAAATCCCAGC

CGAAGCAGTTATTGGTTACTCCGACTTGGAAGGAGATTTTGACGTCGCTGT
TTTACCATTCTCTAATTCCACTAATAACGGTCTGTTGTTTATTAATACTACCAT
TGCTTCTATCGCCGCTAAGGAGGAAGGTGTGTCCCTCGAGAAAAGAGAAG
AAGGTGAACCAAAGTTCGTCAACCAGCACTTGTGTGGTTCCCATTTGGTTG
AGGCTCTGTACTTGGTCTGTGGAGAAAGAGGTTTCTTTTACACCCCAAAGG
CTGCTAAGGGTATCGTTGAGCAATGTTGCACCTCTATTTGTTCCCTGTATCA
GTTGGAAAACTACTGCAACTAA
SEQ ID NO:8

Nucleic acid molecules shown as SEQ ID NO: 14 and SEQ ID NO: 15, in which α-factor(SEQ ID NO: 12) and α-factor-S (SEQ ID NO: 1) were separately fused to the site in the upstream of control 1, were synthesized. EcoR I and Not I restriction sites were also incorporated at both 5' and 3' ends of the synthesized nucleic acid molecules. The synthesized nucleic acid molecules were ligated to the T vector.

ATGAGATTTCCTTCAATTTTTACTGCAGTTTTATTCGCAGCATCCTCCGCATT
AGCTGCTCCAGTCAACACTACAACAGAAGATGAAACGGCACAAATTCCGG
CTGAAGCTGTCATCGGTTACTCAGATTTAGAAGGGGATTTCGATGTTGCTGT
TTTGCCATTTTCCAACAGCACAAATAACGGGTTATTGTTTATAAATACTACTA
TTGCCAGCATTGCTGCTAAAGAAGAAGGGGTATCTCTCGAGAAAAGAGAA
GAAGGTGAACCAAAGTTCGTTAACCAACACTTGTGCGGTTCCCACTTGGTT
GAAGCTTTGTACTTGGTTTGCGGTGAAAGAGGTTTCTTCTACACTCCTAAG
GCTGCTAAGGGTATTGTCGAACAATGCTGTACCTCCATCTGCTCCTTGTACC
AATTGGAAAACTACTGCAACTAA
SEQ ID NO:14

ATGAGATTTCCTTCTATTTTCACTGCTGTTTTGTTTGCCGCTTCCTCTGCTTT
GGCAGCTCCAGTTAATACAACCACTGAAGATGAGACTGCTCAAATCCCAGC
CGAAGCAGTTATTGGTTACTCCGACTTGGAAGGAGATTTTGACGTCGCTGT
TTTACCATTCTCTAATTCCACTAATAACGGTCTGTTGTTTATTAATACTACCAT
TGCTTCTATCGCCGCTAAGGAGGAAGGTGTGTCCCTCGAGAAAAGAGAAG
AAGGTGAACCAAAGTTCGTTAACCAACACTTGTGCGGTTCCCACTTGGTTG
AAGCTTTGTACTTGGTTTGCGGTGAAAGAGGTTTCTTCTACACTCCTAAGG
CTGCTAAGGGTATTGTCGAACAATGCTGTACCTCCATCTGCTCCTTGTACCA
ATTGGAAAACTACTGCAACTAA
SEQ ID NO:15.

The above T vector and the expression vector pPIC3.5K were digested with both endonucleases EcoR I and Not I, and then the target fragment and the vector fragment were separately recovered by Gel Recovery Kit. After purification of the digested fragments, and the target fragment was ligated to the vector pPIC3.5K with T4 ligase.

The above-mentioned ligation solution was transformed into E. coli TOP10 competent cells, and plated onto a plate with ampicillin resistance. After cultivation, the bacterial colony was picked, and the plasmid was extracted and verified by digestion with both restriction enzymes. Four recombinant expression vectors which separately incorporatea-factor or α-factor-S signal peptide for expressing insulin precursor gene with different nucleotide sequences, were finally obtained.

### Example 4 Expression of insulin precursor by Pichia Pastoris recombinant strain, before and after optimization

The recombinant expression vectors constructed in Example 3 were separately transformed into Pichia Pastoris GS115.

The recombinant bacterium colonies were inoculated into 5 mL YPD medium, and cultivated at a constant temperature of 30°C while shaking at 250 rpm, until the value of OD₆₀₀ reached about 10 (16-18 hours). The cells were collected and re-suspended in 50 mL BMGY medium, and cultivated overnight at a constant temperature of 30°C while shaking at 250 rpm, until the value of OD₆₀₀ reached about 30. The cells were collected by centrifuging at 1500 rpm for 5 minutes and re-suspended in 25 mL of BMMY medium. 1/200 volume of methanol (final concentration of 0.5%) was added into the medium, and cultivated at a constant temperature of 30°C while shaking at 250 rpm for 96 hours, while 1/200 volume of methanol was supplemented every 24 hours. Upon the expression was finished, the supernatant was obtained after centrifugation at 10,000 rpm. The yield of the insulin precursor comprised in the supernatant was measured by HPLC.

The recombinant bacterium expressing control 1 gene fused to α-factorwas used as a control bacterium. The yield of insulin precursor by other strains was converted into a percentage relative to the yield of the insulin precursor expressed by the control strain, as shown as Table 2.

**Table 2**

| Bacterial strain | vector | Signal peptide | Expression Gene | Percentage of yield (%) |
|---|---|---|---|---|
| Control bacterium^{.} | pPIC3.5K | α-factor | Control 1 | 100 |
| test bacterium | pPIC3.5K | α-factor-S | Control 1 | 150 |
| test bacterium | pPIC3.5K | α-factor | IP-S | 225 |
| test bacterium | pPIC3.5K | α-factor-S | IP-S | 275 |

The data in Table 2 shows that the yield of the insulin precursor was increased by 1.5 times after merely optimizing the signal peptide in the nucleic acid molecule, and such yield was increased by 2.25 times after merely optimizing the insulin precursor gene. By contrast, the yield of the insulin precursor was increased by 2.75 times after optimizing both the signal peptide and the insulin precursor gene. Taken together, codon optimization can increase the expression level of insulin precursor to 1.5-2.75 times.

## Claims

1. A nucleic acid molecule comprising molecule or structure having the following general formula:
5'- (PS)ₐ - (SP)_{b} - (LS)_{c} - GE -(P'S)_{d} - 3',
wherein PS is a nucleic acid molecule encoding processing site, a is 0 or 1;
SP is a nucleic acid molecule encoding signal peptide, b is 0 or 1;
LS is a nucleic acid molecule encoding spacer peptide, c is 0 or 1;
GE is a nucleic acid molecule encoding polypeptide of interest; and
P'S is a nucleic acid molecule encoding processing site, d is 0 or 1; and
the nucleic acid molecule encoding signal peptide comprises sequence shown as SEQ ID NO:1.

2. A nucleic acid molecule comprising molecule or structure having the following general formula:
5'- (PS)ₐ - (SP)_{b} - (LS)_{C} - GE - (P'S)_{d} - 3',
wherein PS is a nucleic acid molecule encoding processing site, a is 0 or 1;
SP is a nucleic acid molecule encoding signal peptide, b is 1;
LS is a nucleic acid molecule encoding spacer peptide, c is 0 or 1;
GE is a nucleic acid molecule encoding human insulin analogue precursor polypeptide; and
P'S is a nucleic acid molecule encoding processing site, d is 0 or 1; and
the nucleic acid molecule encoding human insulin analogue precursor polypeptide comprises sequence shown as SEQ ID NO:3.

3. The nucleic acid molecule according to claim 1, wherein the polypeptide of interest is human insulin analogue precursor, and the nucleic acid molecule encoding the human insulin analogue precursor comprises a nucleic acid molecule encoding the amino acid sequence shown as SEQ ID NO: 4, preferably, comprises nucleic acid sequence shown as SEQ ID NO: 3.

4. The nucleic acid molecule according to claim 2, wherein the nucleic acid molecule encoding signal peptide comprises a nucleic acid molecule encoding the amino acid sequence shown as SEQ ID NO: 2, preferably comprises nucleic acid sequence shown as SEQ ID NO: 1 or SEQ ID NO: 12.

5. The nucleic acid molecule according to any one of the preceding claims 2 to 4, wherein the nucleic acid molecule encoding human insulin analogue precursor comprises substitution at positions 88-96 of SEQ ID NO: 3, preferably, is substituted with GCCGCTAAG, GCTGCCAAG, GCTGCTAAA or GCCGCCAAG.

6. The nucleic acid molecule according to any one of the preceding claims, wherein the amino acid sequence of the spacer peptide comprises EEGEPK (Glu-Glu-Gly-Glu-Pro-Lys), preferably, the nucleic acid molecule encoding the spacer peptide comprises sequence shown as SEQ ID NO: 5.

7. The nucleic acid molecule according to any one of the preceding claims, comprising any one selected from the group consisting of SEQ ID NO: 1, SEQ ID NO: 3, SEQ ID NO: 6, SEQ ID NO: 8, SEQ ID NO: 13 and SEQ ID NO:15; or consisting of any one selected from the group consisting of SEQ ID NO: 1, SEQ ID NO: 3, SEQ ID NO: 6, SEQ ID NO: 8, SEQ ID NO: 13 and SEQ ID NO:15.

8. The nucleic acid molecule according to any one of the preceding claims, wherein the processing site is restriction site, preferably, PS is a nucleic acid molecule encoding EcoR I restriction site and/or P'S is a nucleic acid molecule encoding Not I restriction site.

9. A vector comprising the nucleic acid molecule according to any one of the preceding claims, preferably the vector is eukaryotic expression vector or prokaryotic expression vector.

10. A host cell comprising the nucleic acid molecule of any one of claims 1-8 and/or the vector of claim 9, preferably the host cell is yeast; more preferably, *Pichia Pastoris.*

11. A method for producing a human insulin analogue comprising the use of the nucleic acid molecule of any one of claims 1-8, the vector of claim 9, and/or the host cell of claim 10.

12. The method of claim 11, further comprising the step of:
1) expressing a human insulin analogue precursor; and
2) obtaining a human insulin analogue by enzymatically digesting the human insulin analogue precursor obtained in step 1).

13. The method according to claim 11 or 12, wherein the human insulin analogue is human insulin with deletion of B30, and/or the human insulin analogue is further substituted with an acylated group; preferably, the lysine at position B29 is substituted with an acylated group; more preferably, the product after substitution is lysine B29 (N^{ε}-(N^{α}-hexadecane fatty diacid-L-lysine-N^{ε}-oxobutylyl)) des(B30) human insulin.
